(19) Europäisches Patentamt
European Patent Office
Office européen des brevets

(11) Publication number: 0 424 616 A2

(12) # EUROPEAN PATENT APPLICATION

(21) Application number: 90114257.0

(51) Int. Cl.[5]: C07C 303/16

(22) Date of filing: 25.07.90

(30) Priority: 24.10.89 US 426343

(43) Date of publication of application: 02.05.91 Bulletin 91/18

(84) Designated Contracting States: BE DE DK FR GB IT NL SE

(71) Applicant: ATOCHEM NORTH AMERICA, INC.
Three Parkway
Philadelphia, Pennsylvania 19102(US)

(72) Inventor: Husain, Altaf
3313 E. Hayes Road
Norristown, Pennsylvania 19401(US)
Inventor: Wheaton, Gregroy Alan
130 Harvest Road
Swedesboro, New Jersey 08085(US)

(74) Representative: Kraus, Walter, Dr. et al
Patentanwälte Kraus, Weisert & Partner
Thomas-Wimmer-Ring 15
W-8000 München 22(DE)

(54) **Production of alkanesulfonic acids by oxidation of alkanethiols and dialkyl disulfides.**

(57) Alkanesulfonic acids are prepared by oxidation of the corresponding alkanethiol or dialkyl disulfide using an oxygen-containing gas as the oxidizing agent, in the presence of water and catalytic amounts of a lower dialkyl sulfoxide and a hydrogen halide.

EP 0 424 616 A2

# PRODUCTION OF ALKANESULFONIC ACIDS BY OXIDATION OF ALKANETHIOLS AND DIALKYL DISULFIDES

## Field of the Invention

The invention relates to the manufacture of alkane sulfonic acids by oxidation of the corresponding alkanethiol or dialkyl disulfide. More specifically, it relates to the oxidation of such compounds using air or an oxygen-containing gas, in the presence of catalytic amounts of a lower dialkyl sulfoxide and a hydrogen halide, and water, to form the corresponding sulfonic acids.

## Background of the Invention

The most commonly used method for the manufacture of alkanesulfonic acids involves oxidation of the corresponding alkanethiol or dialkyl disulfide by chlorine in concentrated hydrochloric acid media (e.g., U.S. Pat. 3,626,004; U.S. Pat. 4,280,966; and EP 040,560). This method suffers from the disadvantage that a significant amount of side product is formed. The side product results from the side chain chlorination of the alkyl group, particularly in the case of higher alkanesulfonic acids. A large amount of hydrochloric acid is also formed as a by-product of the oxidation reaction.

Oxidation of aliphatic thiols and disulfides to the corresponding sulfonic acid by heating in an excess of dimethyl sulfoxide with catalytic amounts of bromine or iodine or their halogen halides is reported by Lowe, J. Org. Chem. , 41(11), 2061-2064 (1976); and U.S. Pat. 3,948,922. Oxidation of sulfide-sulfur bond-containing compounds by lower dialkyl sulfoxides, promoted by bromide ion, is described in U.S. Pat. 3,428,671. Oxidation of thiols to disulfides by their reaction with a lower alkyl sulfoxide in the presence of a catalyst consisting of chlorine or hydrogen chloride as the primary catalyst and iodine or hydrogen iodide as a secondary catalyst is disclosed in U.S. Pat. 3,954,800.

In each of the above examples of oxidation using dimethyl sulfoxide as the oxidizing agent, the latter is consumed in stoichiometric quantities. In view of the high cost of dimethyl sulfoxide, such processes are not considered economic. In addition, the by-product, dimethyl sulfide, may cause severe environmental problems, because of its intense odor.

What is needed is a process for preparing alkane-sulfonic acids which avoids excessive consumption of dialkyl sulfoxide, and avoids the generation of large amounts of side product, particularly dialkyl sulfide.

## Summary of the invention

The invention comprises a process for preparing an alkane sulfonic acid by contacting an alkanethiol, dialkyl disulfide, or a mixture thereof with an oxygen-containing gas in the presence of water and catalytic amounts of a lower dialkyl sulfoxide and a hydrogen halide, to produce the corresponding alkanesulfonic acid.

## Detailed Description of the Invention

We have found that alkanethiols or dialkyl disulfides may be readily converted to their corresponding alkanesulfonic acids where oxygen is utilized as the stoichiometric oxidant and dimethyl sulfoxide is employed only in catalytic amounts. Substantially no side-chain halogenated products are formed in the process of the invention due to the absence of a significant free halogen concentration at any time during the reaction. In addition, no significant free concentration of odorous dimethyl sulfide is present at any time during the reaction.

The process of this invention involves oxidation of an organosulfur reactant selected from alkanethiols and dialkyl disulfides with an oxygen-containing gas such as air, in the presence of water, preferably a large excess thereof, and catalytic amounts of a lower dialkyl sulfoxide and a hydrogen halide. Without wishing to

be bound by any theory, the process is believed characterized by the following reactions:

$$RSH + 3X_2 + 3H_2O \longrightarrow RSO_3H + 6HX$$

$$3R'-\overset{O}{\overset{\|}{S}}-R'' + 6HX \longrightarrow 3R'-S-R'' + 3H_2O + 3X_2$$

$$3R'-S-R'' + 3/2\ O_2 \longrightarrow 3R'-\overset{O}{\overset{\|}{S}}-R''$$

<u>overall:</u>

$$RSH + 3/2\ O_2 \longrightarrow RSO_3H$$

<u>or</u>:

$$RSSR + 5X_2 + 6H_2O \longrightarrow 2RSO_3H + 10HX$$

$$5R'-\overset{O}{\overset{\|}{S}}-R'' + 10HX \longrightarrow 5R'-S-R'' + 5H_2O + 5X_2$$

$$5R'-S-R'' + 5/2\ O_2 \longrightarrow 5R'-\overset{O}{\overset{\|}{S}}-R''$$

<u>overall:</u>

$$RSSR + 5/2\ O_2 + H_2O \longrightarrow 2RSO_3H$$

The alkanethiols that are employed as starting materials in the present invention typically have 1-18 carbon atoms, preferably 1-8 carbon atoms. Thus, there may be used, for example, methanethiol, ethanethiol, n-pro panethiol, isopropanethiol, 1-butanethiol, 2-butanethiol, 1-hexanethiol, 1-octanethiol, 1-decanethiol, and the like.

The dialkyl disulfides which may be used as starting materials typically have 2-24 carbon atoms. They are of the general formula $RSSR'$ where R and $R'$ are alkyl groups which typically may contain 1-12 carbon atoms. R and $R'$ may be the same or different, but are preferably the same. Thus, there may be used as starting material, for example, dimethyl disulfide, diethyl disulfide, dipropyl disulfide, diisopropyl disulfide, dibutyl disulfide, or didecyl disulfide.

The lower dialkyl sulfoxides that are employed are of the general formula $RS(O)R'$ where R and $R'$ are the same or different alkyl groups which may typically contain 1-5 carbon atoms. Dimethyl sulfoxide is the preferred dialkyl sulfoxide because of its ready availability in laboratory as well as commercial scale facilities.

Only a catalytic amount of dialkyl sulfoxide is used in the process of the invention. This is contrasted with the prior art, wherein dialkyl sulfoxide is the oxidizing agent, and is employed in at least stoichiometric amounts with respect to the alkanethiol or dialkyl disulfide starting material. Typically, the amount of dialkyl sulfoxide employed in the process of the invention is not in excess of about 0.25 mole, for each mole of alkanethiol or dialkyl disulfide. Preferably the amount ranges from about 0.05 mole to about 0.25 mole, most preferably, from about 0.1 mole to about 0.2 mole, for each mole of alkanethiol or dialkyl disulfide.

Similarly, only a catalytic amount of hydrogen halide is employed. The amount typically should not exceed about 0.1 mole for each mole of alkanethiol or dialkyl disulfide. The amount of hydrogen halide

used may typically range from about 0.01 mole to about 0.1 mole for each mole of alkanethiol or dialkyl disulfide. Preferably, the amount of hydrogen halide used is from about 0.02 mole to about 0.1 mole for each mole of alkanethiol or dialkyl disulfide.

The hydrogen halide is preferably selected from hydrogen bromide, hydrogen iodide, and mixtures thereof, but it is most preferred that hydrogen bromide is used.

A large excess of water is employed in the process of the invention, generally at least about 4 moles for each mole of alkanethiol, or at least about 6 moles for each mole of dialkyl disulfide. Typically, from about 4 moles to about 100 moles of water per mole of alkanethiol, and from about 6 moles to about 100 moles of water per mole of dialkyl disulfide, are used. It is preferred that the amount of water is from about 10 moles to about 100 moles, per mole of alkanethiol or dialkyl disulfide.

While less than about 4 moles of water may be utilized in the practice of the invention, the reaction product yield is decreased.

The temperature at which the process is carried out can vary, and is preferably about 90° C to about 150° C. Most preferably, the temperature ranges from about 110° C to abut 115° C.

The oxygen-containing gas utilized may be oxygen or a mixture of oxygen and other gases. For example, the oxygen-containing gas may comprise air, a mixture of air and oxygen, or a mixture of oxygen and an inert gas such as, for example, nitrogen, helium, argon, carbon dioxide and mixtures thereof. The concentration of oxygen in the gas mixture is preferably from about 5% to about 50%, by volume, most preferably from about 20% to about 50%. The oxygen-containing gas should not contain any halogen, which leads to unwanted by-product formation.

The pressure at which the process is carried out can vary widely. While pressures from about 100 psig to about 1000 psig may be advantageously employed, preferably, the pressure is from about 125 psig to about 250 psig.

The reaction time is such as to achieve maximum conversion of the alkanethiol or dialkyl disulfide to the corresponding alkanesulfonic acid.

The process of the invention may be carried out in a batchwise or a continuous manner. In the batchwise process, the pressure vessel is charged with the alkanethiol or dialkyl disulfide, dialkyl sulfoxide, hydrogen halide and water, and then pressurized with oxygen-containing gas. The oxygen-containing gas is continuously added throughout the duration of the reaction in order to maintain a desired pressure. The reactor contents are advantageously vigorously agitated to give maximum mixing of the reactants. Alternatively, either the liquid or gas, or both, may be recirculated in order to produce maximum mixing.

In the continuous mode of reaction, fresh liquid and gas feed is added to the reactor at the desired pressure. The reaction mixture containing the product alkanesulfonic acid is removed continuously to maintain a constant reactor liquid level, and to provide the desired reactant residence time. The reactor contents are vigorously agitated. The alkanesulfonic acid is then separated from the unreacted alkanethiol or dialkyl disulfide, and the catalysts may be recycled to the reactor.

The process of the invention is illustrated by, but not limited to, the following examples.

## Example 1

n-Propanethiol (0.52 g; 6.8 mmole), dimethyl sulfoxide (0.1 ml; 1.5 mmole), hydrogen iodide (0.02 ml of a 47 wt.% aqueous solution; 0.13 mmole) and water (10 ml) were charged into a glass pressure reactor. The reactor was closed, pressurized with air to about 100 psig, and heated to 110° C. The reactor pressure at this temperature was about 150 psig. The reactor was kept at the same temperature for 24 hours while the contents were stirred, after which time the reactor was cooled and slowly depres surized. The contents of the reactor were extracted with 25 ml of water. In this manner 0.4 g (47% yield) of n-propane sulfonic acid was produced as determined by titration of the aqueous fraction by 0.1 Normal aqueous sodium hydroxide.

## Example 2

n-Propanethiol (0.52 g; 6.8 mmole), dimethyl sulfoxide (0.1 ml; 1.5 mmole), hydrogen bromide (0.12 ml of a 48 wt.% aqueous solution; 0.6 mmole) and water (10 ml) were charged into a glass pressure reactor. The reactor was closed and pressurized with 45 psig oxygen and 65 psig air to a total of about 110 psig.

The reactor was heated to 110°C. The reactor pressure at this temperature was about 150 psig. The reactor was kept at this temperature for 24 hours while stirring the contents with a magnetic stirrer. After this time the reactor was cooled and slowly depressurized. The contents of the reactor were extracted with 25 ml of water. In this manner, 0.47 g (55% yield) of n-propane sulfonic acid was produced as determined by titration of the aqueous fraction by 0.1 Normal aqueous sodium hydroxide.

The following example illustrates the preference for excess water in the practice of the invention. While the desired sulfonic acid is produced if less than an excess of water is utilized, the amount of product is very small.

## Example 3

n-Propanethiol (25.2 g; 330 mmole), dimethyl sulfoxide (5.8 ml; 80 mmole), hydrogen iodide (0.9 ml of a 48 wt.% aqueous solution; 5.7 mmole) and water 4.0 ml (220 mmole) were charged into a glass pressure reactor. The reactor was closed and pressurized with 100 psig air. The reactor was heated to 110°C. The reactor pressure at this temperature was about 150 psig. The reactor was kept at this temperature for 24 hours while stirring the contents with a magnetic stirrer. The reactor was thereafter cooled and slowly depressurized. The contents of the reactor were extracted with 25 ml of water. In this manner 0.14 g (0.34% yield) of the n-propane sulfonic acid was produced as determined by titration of the aqueous fraction by 0.1 Normal aqueous sodium hydroxide.

The present invention may be embodied in other specific forms without departing from the spirit or essential attributes thereof and, accordingly, reference should be made to the appended claims, rather than to the foregoing specification, as indicating the scope of the invention.

## Claims

1. A process for preparing an alkanesulfonic acid comprising contacting an alkanethiol, dialkyl disulfide or a mixture thereof with an oxygen-containing gas in the presence of water and catalytic amounts of a lower dialkyl sulfoxide and a hydrogen halide, to produce the corresponding alkanesulfonic acid.

2. The process of claim 1, wherein the alkanethiol contains 1-18 carbon atoms and the dialkyl disulfide contains 2-24 carbon atoms.

3. The process of claim 2, wherein water is present in the amount of at least about 4 moles for each mole of alkanethiol and at least about 6 moles for each mole of dialkyl disulfide.

4. The process of claim 2, wherein the oxygen-containing gas comprises air.

5. The process of claim 2, wherein the oxygen-containing gas comprises a mixture of air and oxygen.

6. The process of claim 2, wherein the oxygen-containing gas comprises a mixture of oxygen and an inert gas.

7. The process of claim 6, wherein the inert gas is selected from the group of nitrogen, helium, argon, carbon dioxide and mixtures thereof.

8. The process of claim 2, wherein the concentration of oxygen in said oxygen-containing gas comprises from about 5% to about 50%, by volume.

9. The process of claim 8, wherein the concentration of oxygen in said oxygen-containing gas comprises from about 20% to about 50%, by volume.

10. The process of claim 2, wherein the hydrogen halide is selected from hydrogen bromide, hydrogen iodide, and mixtures thereof.

11. The process of claim 2, wherein the temperature is from about 90°C to 150°C.

12. The process of claim 11, wherein the pressure is from about 100 psig to about 1000 psig.

13. The process of claim 2, wherein the dialkyl sulfoxide comprises dimethyl sulfoxide.

14. The process of claim 2, wherein the lower dialkyl sulfoxide is present in an amount from about 0.05 mole to about 0.25 mole, for each mole of alkanethiol or dialkyl disulfide.

15. The process of claim 14, wherein the lower dialkyl sulfoxide is present in an amount from about 0.1 mole to about 0.2 mole, for each mole of alkanethiol or dialkyl disulfide.

16. The process of claim 14, wherein the hydrogen halide is present in an amount from about 0.01 mole to about 0.1 mole, for each mole of alkanethiol or dialkyl disulfide.

17. The process of claim 14, wherein the amount of water present is from about 4 to about 100 moles per mole of alkanethiol and from about 6 to about 100 moles per mole of dialkyl disulfide.

18. The process of claim 17, wherein the dialkyl sulfoxide comprises dimethyl sulfoxide.

19. A process for preparing an alkanesulfonic acid comprising oxidizing the corresponding alkanethiol, dialkyl disulfide, or mixtures thereof with an oxygen-containing gas in the presence of the following:

(a) for each mole of alkanethiol or dialkyl disulfide:

(i) from about 0.05 to about 0.25 mole lower dialkyl sulfoxide;

(ii) from about 0.01 mole to about 0.1 mole of hydrogen bromide, hydrogen iodide or a mixture thereof;

(b) for each mole of alkanethiol:

(i) from about 4 moles to about 100 moles of water;

(c) for each mole of dialkyl disulfide:

(i) from about 6 moles to about 100 moles of water.

20. A process according to claim 19, wherein the temperature is from about 90° C to about 150° C.

21. A process according to claim 19, wherein the oxygen-containing gas comprises from about 5% to about 50% oxygen, by volume.

22. A process according to claim 19, wherein the lower dialkyl sulfoxide comprises dimethyl sulfoxide.